# FASCICULE DE BREVET EUROPEEN

(11) **EP 1 399 111 B1**
(45) Date de publication et mention de la délivrance du brevet: **14.02.2007**
(21) Numéro de dépôt: 02748952.5
(22) Date de dépôt: 13.06.2002
(51) Int. Cl.: A61K 8/65, A61K 8/73, A61K 8/89, A61Q 1/02, A61Q 19/08

(54) **COMPOSITION STERILE INJECTABLE A VISEE ESTHETIQUE ET/OU REPARATRICE**
INJIZIERBARE STERILE ZUSAMMENSETZUNG ALS SCHÖNHEITS- UND/ODER KORREKTURZWECK
STERILE INJECTABLE COMPOSITION FOR AESTHETIC AND/OR REPARATIVE USES

(30) Priorité: 18.06.2001 FR 0107981
(43) Date de publication de la demande: 24.03.2004
(73) Titulaire: Bellity, Philippe, 92000 Neuilly sur Seine (FR); IOLTECH S.A., 17180 Perigny (FR)
(72) Inventeur: Bellity, Philippe, 92000 Neuilly sur Seine (FR); IOLTECH S.A., 17180 Perigny (FR)
(74) Mandataire: Peaucelle, Chantal
(86) Numéro de dépôt international: PCT/FR2002/002033
(87) Numéro de publication internationale: WO 2002/102328

(56) Documents cités:
- WO-A-00/51553
- US-A- 5 383 930

## Description

L'invention se rapporte à une nouvelle composition stérile injectable à visée esthétique et/ou réparatrice ainsi qu'à l'utilisation d'un appareil mélangeur pour préparer ladite composition. Elle vise également son utilisation dans une méthode de traitement esthétique et/ou réparatrice.

La peau présente souvent de nombreuses imperfections telles que des taches, des dépigmentations, des marques de veines visibles à travers la peau, des cernes profondes, des cicatrices... Ces différences de couleurs, ces anomalies de la pigmentation sont souvent jugées inesthétiques. La pratique quotidienne de la chirurgie esthétique et réparatrice a permis à l'inventeur de constater une demande croissante pour ce type d'intervention réparatrice. Jusqu'à aujourd'hui, cette demande n'était pas satisfaite, faute de moyens appropriés pour compenser les problèmes de différences de couleurs de la peau.

L'inventeur a constaté que de telles marques, considérées généralement comme disgrâcieuses, pouvaient être masquées grâce à l'injection sous-cutanée, sous-dermique et/ou intra-dermique d'une composition stérile d'une combinaison de produits déterminée.

L'invention a donc pour but de fournir une composition stérile injectable capable de redonner à la peau l'esthétique recherchée. Elle propose également l'utilisation d'un appareil mélangeur permettant de fabriquer ladite composition.

Elle vise de plus, une méthode de traitement desdites imperfections permettant, par maquillage interne, de redonner à la peau l'aspect souhaité par le sujet.

Selon la présente invention, la composition stérile injectable à visée esthétique et/ou réparatrice est caractérisée en ce qu'elle comporte, en quantité efficace, au moins un colorant de couleur appropriée et un vecteur biocompatible permettant de contrôler la diffusion dudit colorant, lors de l'injection par nappage sous-cutané, sous-dermique et/ou intra-dermique de ladite composition, précisément sur la zone à traiter.

On appelle vecteur biocompatible, tout vecteur qui peut être injecté, sans danger pour la santé, dans une partie du corps humain, et qui est toléré par celle-ci.

En particulier, le vecteur d'une telle composition comporte au moins un gel, de préférence biorésorbable, permettant au colorant de ne pas migrer. Ce gel est choisi préférentiellement dans le groupe comprenant un acide hyaluronique et/ou un collagène. Un exemple de vecteur approprié non biorésorbable est donné par la silicone. De tels gels, résorbables ou non, sont introduits dans la composition en quantité suffisante afin de donner à la composition une viscosité suffisante pour laisser en place le maquillage interne, une fois l'injection effectuée. En effet, plus la composition est visqueuse, plus sa localisation sera précise. En revanche, une viscosité trop importante peut induire une déformation de la peau. C'est pourquoi, pour des zones de peau fine, telles que la paupière par exemple, il est nécessaire d'utiliser une composition relativement fluide afin d'obtenir un nappage régulier sous le derme. La proportion de vecteur à incorporer résulte donc d'un compromis sur la viscosité. Ces vecteurs permettent ainsi un contrôle complet de la localisation du colorant tant lors de l'injection que dans le temps.

Le colorant de cette composition est choisi en fonction de la carnation de la peau à traiter. Sa teinte est identique à celle de la peau. Il est préférable de jouer sur la dilution du colorant dans la composition pour définir l'intensité de la coloration. Pour être utilisable à des fins d'injection, le colorant doit présenter des garanties de sécurité sanitaire. La proportion de colorant à introduire dans la composition selon l'invention, varie en fonction du degré de transparence de la peau du sujet à traiter. En général, plus la peau est transparente, plus la dilution est importante.

La composition de l'invention est stérile car elle est destinée à être injectée de manière sous-cutanée, sous-dermique et/ou intra-dermique afin de former un écran sous le derme, sous l'épiderme et/ou dans le derme.

L'invention propose également l'utilisation d'un appareil mélangeur capable de préparer le colorant de la composition selon l'invention après analyse de la coloration de la peau du sujet à traiter. Plus particulièrement, l'appareil utilisé comporte un colorimètre afin de reconstituer une teinte spécifique, à partir d'une gamme de colorants, par mélange de ceux-ci, dans des proportions calculées par l'appareil mélangeur. L'appareil utilisé peut, d'autre part, combiner avantageusement un colorimètre et un viscosimètre de manière à préparer extemporanément la composition selon l'invention. Le choix du vecteur et la viscosité finale de la composition est définie par le praticien en fonction des paramètres cités précédemment. L'appareil peut alors, en fonction de ces données, fabriquer une composition ayant la teinte et la viscosité recherchées. Enfin, l'utilisation de l'appareil selon l'invention peut être stérile afin que la composition préparée soit utilisable de suite.

L'invention vise aussi à fournir une méthode de traitement esthétique par maquillage interne de la peau caractérisée en ce qu'on effectue une injection sous-cutanée, sous-dermique et/ou intra-dermique de la composition selon l'invention afin de créer un écran opaque localisé tout en laissant intacte la transparence des couches supérieures de l'épiderme. Le choix du type d'injection dépend de la finesse de la peau à traiter. On utilise en particulier une injection intra-dermique pour le maquillage d'une cicatrice, tandis que le maquillage d'une cerne est effectuée par une injection sous-dermique. L'injection est donc effectuée à une profondeur donnée, dépendante de la zone à traiter, de l'épaisseur et de la transparence de la peau. De même, la quantité de produit à injecter ainsi que le nombre d'injections dépendent de l'étendue de la zone à traiter et de sa localisation sur le corps.

L'avantage d'une telle méthode est de pouvoir corriger les imperfections de la peau en transparence. En effet, les injections étant localisées en profondeur, par nappage sous-cutané, sous-dermique et/ou intra-dermique de la zone à traiter à l'aide de la composition, l'aspect transparent des couches supérieures de l'épiderme est préservé. Cette méthode permet donc d'obtenir un résultat très naturel.

En particulier, une telle méthode permet d'éclaicir ou de foncer une zone de peau déterminée. Elle permet aussi de masquer l'aspect noir des cernes par teinture sous-dermique, de recolorer des taches de dépigmentation ou de masquer des veines.

Par exemple, il est fréquent que des veines proches de la surface de la peau laissent apparaître une marque bleue sur celle-ci. L'injection de la composition entre la surface de la peau et la veine forme une sorte d'écran coloré et opaque faisant disparaître de telles marques.

Cette méthode permet enfin de maquiller une cicatrice présentant un aspect inesthétique dû à un problème de coloration.

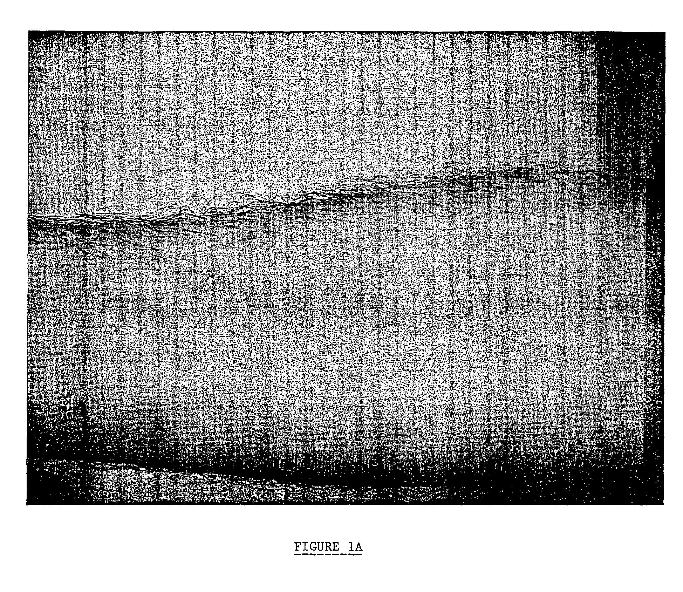

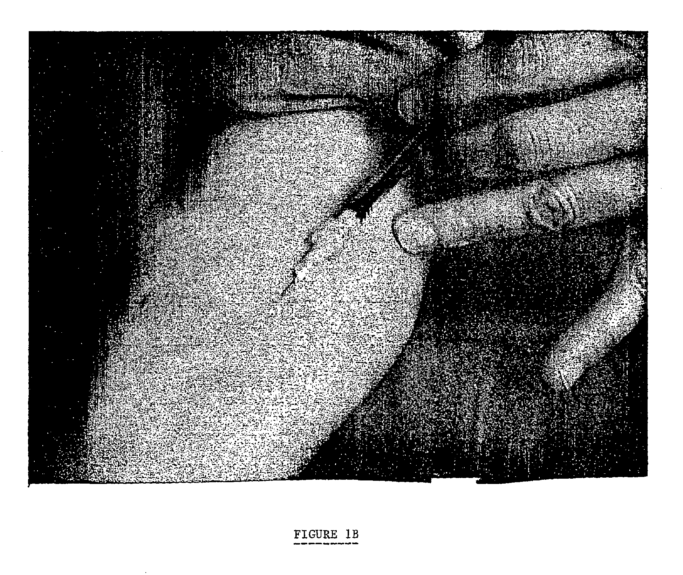

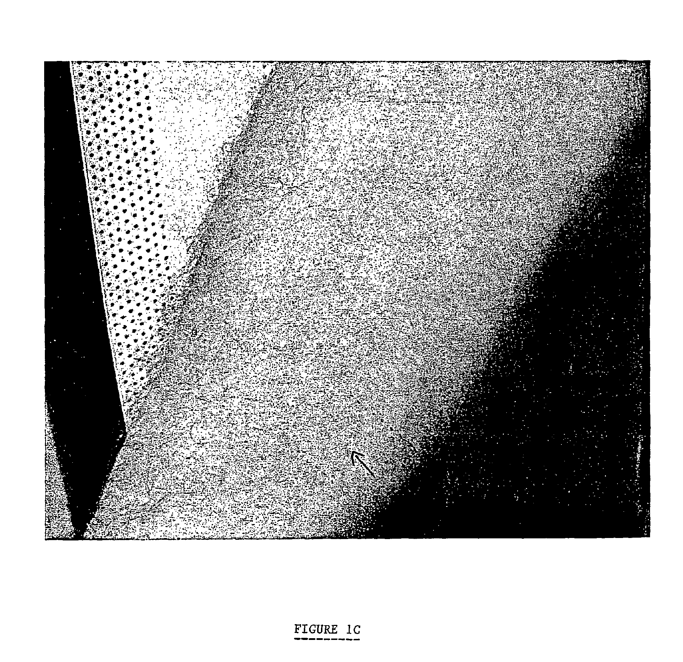

La figure 1 illustre le maquillage d'une veine apparente grâce à l'invention.

D'autres caractéristiques et avantages de l'invention sont donnés dans les exemples suivants.

### Exemple 1: Opacification en profondeur de la peau visant à faire disparaître une marque de veine chez un sujet à peau claire.

### 1. Préparation de la composition

- exemple de composition:
   - 30% de colorant
   - 70% d'acide hyaluronique
- Conditions de préparation:
   Les produits utilisés pour la préparation de la composition selon l'invention sont déjà stériles. Le mélange est effectué sous atmosphère stérile. Cependant, il est possible de stériliser les produits par rayonnement gamma.
   La composition est ensuite conditionnée dans des ampoules stériles.

### 2. Injection de la composition

Il s'agit de masquer une veine située sur l'intérieur du bras. L'injection est donc effectuée de manière sous-dermique. La quantité injectée est d'environ 1 cm³. L'injection est faite en une seule piqûre, l'aiguille étant mobile pour répartir le produit (manière d'opérer identique à celle utilisée pour réparer des rides).

### 3. Résultat

Les figures 1A et 1C montre l'emplacement de la veine sur la bras du sujet, avant l'injection (1A) et trois mois après l'injection (1C). On peut constater la disparition de la marque de la veine. On notera que la teinte de la composition utilisée se fond bien dans la carnation du sujet. La figure 1B illustre l'injection de la composition.

### Exemple 2: Utilisation de l'appareil mélangeur.

L'appareil mélangeur comporte un colorimètre. Dans un premier temps, celui-ci est étalonné à l'aide d'une gamme de couleurs de peau. Une telle gamme est disponible dans le commerce.

Une zone de peau du sujet est soumise au colorimètre qui analyse et détermine les proportions de chacun des colorants de la gamme d'étalonnage afin de reconstituer la couleur de ladite zone de peau. Le praticien entre ensuite dans l'appareil mélangeur les données suivantes:
- le choix du vecteur (collagène, acide hyaluronique...)
- sa viscosité.

Le colorant est alors mélangé avec le vecteur et un solvant approprié. Durant l'opération, le viscosimètre contrôle le respect des données.

La composition est délivrée dans une ampoule après stérilisation.

Une version simplifiée de l'appareil mélangeur comporte un choix d'acides hyaluroniques à des viscosités prédéfinies. Il suffit alors de choisir le type d'acide hyaluronique à mélanger à la composition.

## Revendications

1. Composition stérile injectable à visée esthétique et/ou réparatrice, **caractérisée en ce qu'**elle comporte, en quantité efficace, au moins un colorant opaque de teinte identique à celle de la peau à traiter et un vecteur biocompatible permettant de contrôler la diffusion dudit colorant, lors de l'injection par nappage sous-cutané, sous-dermique et/ou intra-dermique de ladite composition, précisément sur la zone à traiter,

2. Composition selon la revendication 1, **caractérisée en ce que** le vecteur comporte au moins un gel, de préférence biorésorbable, permettant au colorant de ne pas migrer.

3. Composition selon la revendication 2, **caractérisée en ce que** ledit gel est choisi dans le groupe comprenant un acide hyaluronique, un collagène et/ou une silicone.

4. Composition selon selon une quelconque des revendications 1 à 3, **caractérisée en ce que** ledit vecteur est introduit en quantité suffisante dans la composition afin de lui conférer une viscosité lui permettant de rester en place sans déformer la zone traitée.

5. Utilisation d'un colorimètre-mélangeur afin de fabriquer instantanément le colorant de la composition selon l'une quelconque des revendications 1 à 4 à partir d'une gamme de colorants, **caractérisée en ce que** le colorimètre est combiné à un viscosimètre, permettant de fabriquer extemporanément le mélange vecteur-colorant de la composition selon l'une quelconque des revendications 1 à 4.

6. Utilisation selon la revendication 5, **caractérisée en ce qu'**elle permet la fabrication stérile de la composition selon les revendications 1 à 4.

7. Méthode de traitement esthétique et/ou réparatrice par maquillage interne **caractérisée en ce qu'**on effectue une infection sous-cutanée, sous-dermique et/ou intra-dermique de la composition selon l'une quelconque des revendications 1 à 4 afin de créer un écran opaque localisé tout en laissant intact la transparence des couches supérieures de l'épiderme.

8. méthode selon la revendication 7, pour corriger des zones de transparence de la peau.

9. méthode selon la revendication 7, permettant d'éclaircir ou de foncer une zone de peau déterminée.

10. méthode selon la revendication 7, pour masquer des cernes, des veines et/ou des taches de dépigmentation.

11. méthode selon la revendication 7, pour maquiller une cicatrice.

## Claims

1. Sterile injectable composition for aesthetic and/or reparative use, **characterized in that** it comprises, in an effective quantity, at least one opaque dye of a shade identical to that of the skin to be treated and a biocompatible vector making it possible to control the diffusion of said dye on injecting said composition into the sub-cutaneous, sub-dermal and/or intradermal layer precisely in the zone to be treated.

2. Composition according to claim 1, **characterized in that** the vector comprises at least one, preferably bioresorbable, gel which prevents migration of the dye.

3. Composition according to claim 2, **characterized in that** said gel is chosen from the group comprising a hyaluronic acid, a collagen and/or a silicone.

4. Composition according to any one of claims 1 to 3, **characterized in that** said vector is introduced in a sufficient quantity into the composition in order to.give it a viscosity which allows it to remain in place without deforming the treated zone.

5. Use of a colorimeter-mixer in order to instantaneously produce the dye of the composition according to any one of claims 1 to 4 from a range of dyes, **characterized in that** the colorimeter is combined with a viscosimeter making it possible to extemporaneously produce the vector-dye mixture of the composition according to any one of claims 1 to 4.

6. Use according to claim 5, **characterized in that** it allows the sterile production of the composition according to claims 1 to 4.

7. Method of aesthetic and/or reparative treatment by internal correction of imperfections **characterized in that** a sub-cutaneous, sub-dermal and/or intra-dermal injection of the composition according to any one of claims 1 to 4 is carried out in order to create a localized opaque screen while leaving the transparency of the upper layers of the epidermis intact.

8. Method according to claim 7 for correcting transparent areas of the skin.

9. Method according to claim 7 allowing a determined area of skin to be lightened or darkened.

10. Method according to claim 7 for masking circles under the eyes, veins and/or depigmentation spots.

11. Method according to claim 7 in order to disguise a scar.

## Patentansprüche

1. Injizierbare sterile Zusammensetzung zu ästhetischen und/oder Ausbesserungszwecken, **dadurch gekennzeichnet, dass** sie in wirksamer Menge mindestens einen opaken Farbstoff mit einem Farbton, welcher mit demjenigen der zu behandelnden Haut identisch ist, und einen biokompatiblen Vektor aufweist, der das Kontrollieren der Diffusion des Farbstoffs bei der Injektion durch subkutanes, subdermales und/oder intradermales Auftragen der Zusammensetzung genau auf die zu behandelnde Zone ermöglicht.

2. Zusammensetzung nach Anspruch 1, **dadurch gekennzeichnet, dass** der Vektor mindestens ein Gel, vorzugsweise ein biologisch resorbierbares Gel, aufweist, welches das Wandern des Farbstoffs verhindert.

3. Zusammensetzung nach Anspruch 2, **dadurch gekennzeichnet, dass** das Gel aus der Gruppe gewählt ist, die aus einer Hyaluronsäure, einem Kollagen und/oder einem Silikon besteht.

4. Zusammensetzung nach einem der Ansprüche 1 bis 3, **dadurch gekennzeichnet, dass** der Vektor in ausreichender Menge in die Zusammensetzung eingebracht ist, um dieser eine Viskosität zu vermitteln, welche es dieser ermöglicht, an ihrer Position zu verbleiben, ohne die behandelte Zone zu verformen.

5. Verwendung eines Kolorimeters/Mischers zur sofortigen Herstellung des Farbstoffs der Zusammensetzung nach einem der Ansprüche 1 bis 4, ausgehend von einer Farbstoffpalette, **dadurch gekennzeichnet, dass** das Kolorimeter mit einem Viskosimeter kombiniert ist, wodurch die unmittelbare Herstellung der Mischung aus Vektor und Farbstoff der Zusammensetzung nach einem der Ansprüche 1 bis 4 möglich ist.

6. Verwendung nach Anspruch 5, **dadurch gekennzeichnet, dass** sie die sterile Herstellung der Zusammensetzung der Ansprüche 1 bis 4 ermöglicht.

7. Verfahren zur ästhetischen und/oder Ausbesserungsbehandlung durch inneres Kaschieren, **dadurch gekennzeichnet, dass** die Zusammensetzung nach einem der Ansprüche 1 bis 4 subkutan, subdermal und/oder intradermal injiziert wird, um eine lokale opake Abdeckung zu erzeugen, wobei gleichzeitig die Transparenz der oberen Schichten der Epidermis unbeschadet bleibt.

8. Verfahren nach Anspruch 7 zum Korrigieren von Transparenzzonen der Haut.

9. Verfahren nach Anspruch 7, das ein Aufhellen oder Abdunkeln einer bestimmten Hautzone ermöglicht.

10. Verfahren nach Anspruch 7 zum Abdecken von Augenringen, Venen und/oder depigmentierter Flecken.

11. Verfahren nach Anspruch 7 zum Kaschieren einer Narbe.
